(19) Europäisches Patentamt
European Patent Office
Office européen des brevets

(11) Veröffentlichungsnummer: **0 078 949**
**B1**

# (12) EUROPÄISCHE PATENTSCHRIFT

(45) Veröffentlichungstag der Patentschrift: **19.06.85**

(51) Int. Cl.⁴: **C 07 D 405/04**, C 07 D 405/14, A 61 K 31/47 // C07D307/62

(21) Anmeldenummer: **82109713.6**

(22) Anmeldetag: **21.10.82**

(54) **Neue 1-Furyl-3,4-dihydroisochinoline diese enthaltende Arzneimittel sowie Verfahren zu deren Herstellung und deren Verwendung.**

(30) Priorität: **05.11.81 DE 3143876**

(43) Veröffentlichungstag der Anmeldung: **18.05.83 Patentblatt 83/20**

(45) Bekanntmachung des Hinweises auf die Patenterteilung: **19.06.85 Patentblatt 85/25**

(84) Benannte Vertragsstaaten: **AT BE CH DE FR IT LI LU NL SE**

(56) Entgegenhaltungen:
**EP - A - 0 037 934**
**DE - A - 2 722 773**

**Die Akte enthält technische Angaben, die nach dem Eingang der Anmeldung eingereicht wurden und die nicht in dieser Patentschrift enthalten sind.**

(73) Patentinhaber: **BOEHRINGER INGELHEIM KG, D-6507 Ingelheim am Rhein (DE)**

(72) Erfinder: **Lösel, Walter, Dr., Im Herzenacker 26, D-6535 Gau-Algesheim (DE)**
Erfinder: **Roos, Otto, Dr., Elsheimer Strasse 36, D-6501 Schwabenheim (DE)**
Erfinder: **Reichl, Richard, Dr., Im Hippel 55, D-6535 Gau-Algesheim (DE)**
Erfinder: **Kuhn, Franz-Josef, Dr., Beethovenstrasse 11, D-6535 Gau-Algesheim (DE)**

**EP 0 078 949 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf 1-Furyl-3,4-dihydroisochinoline der allgemeinen Formel I,

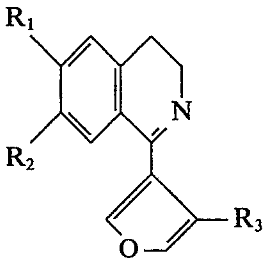

(I)

worin

$R_1$ eine Methoxygruppe,
$R_2$ eine Methoxy- oder Hydroxygruppe,
$R_3$ eine Cyanogruppe oder eine

$\underset{\underset{O}{\|}}{-C}-Y$-Gruppe

bedeutet, wobei

Y —OH, —O-Alkyl mit 1 bis 3 Kohlenstoffatomen,

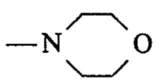, $-N(CH_3)_2$, $-N(C_2H_5)_2$,

—NH-Alkyl mit 1 bis 5 Kohlenstoffatomen,
$-NH-CH_2-CHOH-CH_3$, $-NH-CH_2-C\equiv CH$

oder eine Gruppe der Teilformel

$-NH-(CH_2)_n-R$

bedeutet, in der

n die Zahlen 1 bis 3 und
R $-N(CH_3)_2$, $-OCH_3$, $-Cl$,

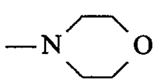, 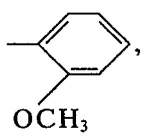, 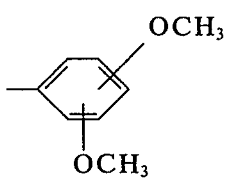 oder 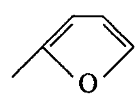

bezeichnen und deren Säureadditionssalze.

Bevorzugt sind 1-Furyl-3,4-dihydroisochinoline der allgemeinen Formel

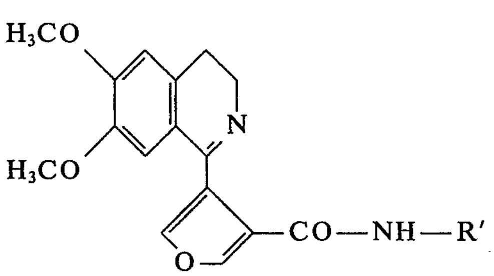

(Ia)

worin R' eine Alkylgruppe mit 1 bis 5 Kohlenstoffatomen, eine Dialkylaminoalkylgruppe oder den Rest —CN bedeutet und deren Säureadditionssalze.

Besonders bevorzugt sind 1-Furyl-3,4-dihydroisochinoline der allgemeinen Formel Ia, worin R' eine Alkylgruppe der Teilformel

$-(CH_2)_2-CH_3$, $-(CH_2)_3-CH_3$ oder $-CH_2-CH(CH_3)_2$

bedeutet und deren Säureadditionssalze.

Die neuen Verbindungen können nach folgenden Verfahren hergestellt werden.

a) durch Cyclisierung eines Amids der Furan-3,4-dicarbonsäure der allgemeinen Formel

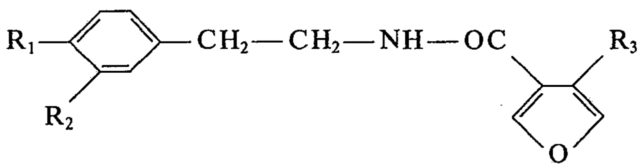

(II)

worin $R_1$ bis $R_3$ die oben genannten Bedeutungen besitzen, mit Lewissäuren.

Als Konditionsmittel eignen sich insbesondere Lewissäuren wie Phosphortrichlorid, Phosphorpentachlorid, Phosphoroxychlorid, Bortrifluorid, Zinntetrachlorid aber auch starke Mineralsäuren, wie Schwefelsäure, Fluorsulfonsäure, Fluorwasserstoffsäure oder Polyphosphonsäure. Sie werden gewöhnlich im Überschuß eingesetzt. Bevorzugt wird Phosphoroxychlorid.

Die Cyclisierung kann in Gegenwart oder in Abwesenheit eines Lösungsmittels durchgeführt werden. Geeignet sind alle innerten Lösungsmittel, soweit sie eine ausreichende Löslichkeit für die Reaktionspartner besitzen und einen ausreichend hohen Siedepunkt aufweisen. Beispiele sind Benzol, Alkylbenzole, Chlorbenzole, Dekalin, Chloroform, Methylenchlorid, Acetonitril und dergleichen. Eine Variante des Verfahrens besteht darin, daß Kondensationsmittel beispielsweise Phosphoroxychlorid selbst als Lösungsmittel zu verwenden.

Bezüglich der Reaktionstemperatur bestehen keine speziellen Bedingungen. Die erfindungsgemäße Umsetzung kann innerhalb eines großen Temperaturbereichs, vorzugsweise unter Erwärmen oder Erhitzen bis zum Siedepunkt des Lösungsmittels durchgeführt werden.

b) durch Umsetzung eines 3-[(3,4-Dihydroisochinolyl-(1)]-furan-4-carbonsäurederivat der allgemeinen Formel

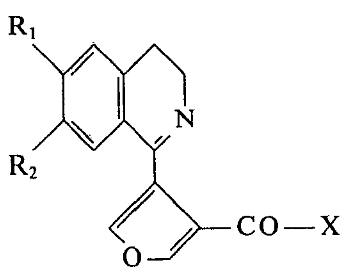

(III)

worin $R_1$ und $R_2$ die oben genannten Bedeutungen besitzen und X ein Chloratom oder eine Methoxy- oder Ethoxygruppe oder eine Imidazolidgruppe bedeutet, mit einer Verbindung der allgemeinen Formel

$Y-H$ (IV)

worin Y wie vorstehend angegeben definiert ist.

Dieses Verfahren beinhaltet unter anderem die Umwandlung von 1-Furyl-3,4-dihydroisochinolinen der Formel I und Ia in andere Endprodukte. So können entsprechende Carbonsäureester oder Carbonsäureamide durch Verseifung mit starken Alkalien in entsprechende 3-Dihydroisochinolyl-(1)-furan-4-carbonsäuren der allgemeinen Formel I umgewandelt werden. Weiterhin bietet dieses Verfahren die Möglichkeit, Säurechloride, Ester und Imidazolide der Formel III mit geeigneten Aminen, die der Formel IV entsprechen, zu den Verbindungen der Formeln I und Ia umzusetzen.

Die Ausgangsverbindungen der Formel II stellen zum Teil neue Verbindungen dar und werden in der Weise hergestellt, daß man ein geeignetes reaktionsfähiges Derivat der Furan-3,4-dicarbonsäure mit einem primären oder sekundären Amin direkt zu Verbindungen der Formel II umsetzt oder aber zunächst ein Monoamidderivat der Furan-3,4-dicarbonsäure herstellt und dieses als solches oder indem man es nochmals in eine reaktive Form überführt, mit einem weiteren Amin in der Weise umsetzt, daß ein Derivat entsteht, das der Formel II entspricht.

Für die Herstellung der Ausgangsverbindungen der allgemeinen Formel II sind beispielsweise solche Furan-3,4-dicarbonsäurederivate geeignet, bei denen die Carboxylgruppen in Säurehalogenide, -azide

oder gemischte Anhydride, z. B. mit einer aromatischen oder aliphatischen Carbonsäure, Alkylkohlensäure oder Dialkylphosphorsäure überführt sind oder aber in Form aktiver Säureamide, beispielsweise mit Imidazol, 4-substituiertem Imidazol, Dimethylpyrazol, Triazol oder Tetrazol oder als aktive Ester, z. B. Cyanmethyl-, Methoxymethyl-, Vinyl-, Propargyl-, Nitrophenyl-, Methansulfonyl-, Pyridylalkylester oder als Ester mit Dimethylhydroxylamin, 1-Hydroxysuccinimid, 1-Hydroxybenztriazol, Dicyclohexylharnstoff usw. vorliegen. Bevorzugt wird das Furan-3,4-dicarbonsäuremonoethylesterchlorid.

Die Amidbildung wird in der Regel in einem Lösungsmittel wie Dioxin, Acetonitril, Tetrahydrofuran, Pyridin oder irgend einem anderen organischen Lösungsmittel, welches die Reaktion nicht nachteilig beeinflußt, gegebenenfalls in Gegenwart einer organischen oder anorganischen Base als Säurefänger durchgeführt. Diese Lösungsmittel können auch in Form einer Mischung verwendet werden. Es ist aber auch möglich, das Amin im Überschuß eingesetzt, als Lösungsmittel zu verwenden.

Die Reaktionstemperatur ist nicht kritisch und die Umsetzung wird in der Regel unter Kühlen, bei Umgebungstemperatur, unter Erwärmen oder unter Erhitzen vorgenommen.

Ausgangsverbindungen der Formel III können aus den entsprechenden 3-[(3,4-Dihydroisochinolyl-(1)]-furan-4-carbonsäuren hergestellt werden.

Die erfindungsgemäßen 1-Furyl-3,4-dihydroisochinoline der allgemeinen Formel I sind Basen und können auf übliche Weise in beliebige, physiologisch unbedenkliche mit anorganischen oder organischen Säuren gebildete Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, 8-Chlortheophyllin, Methansulfonsäure und dergleichen.

Die neuen 1-Furyl-3,4-dihydroisochinoline der allgemeinen Formel I besitzen sowohl als Basen als auch in Form ihrer Salze im Tierversuch wertvolle therapeutische Eigenschaften. Insbesondere verbessern sie die Gewebsdurchblutung und Gewebssauerstoffversorgung besonders im zentralen Nervensystem. Zudem verfügen sie über eine kontraktilitätssteigernde und blutdruckbeeinflussende Wirkkomponente. Für die angegebene Indikation stellen die Verbindungen eine neue Stoffklasse dar; sie können sich daher besonders in jenen Fällen als wertvoll erweisen, in denen die herkömmlichen im Handel befindlichen Arzneimittel versagen. Darüber hinaus stellen die beschriebenen Verbindungen vielseitig abwandelbare Zwischenprodukte dar.

Die Toxizitäten sind im allgemeinen gering, wie durch Toxizitätsuntersuchungen an der Maus bei i. v. und p. o.-Gabe festgestellt werden konnte.

Aus der EP-A-37 934 sind $\alpha$-Aminocarbonyl-1-benzyl-3,4-dihydro-isochinoline mit der gleichen Indikation bekanntgeworden. Diese Verbindungen haben jedoch mit den Verbindungen der vorliegenden Anmeldung lediglich den Isochinolinring gemeinsam; er ist hier mit dem $\alpha$-C-Atom der Benzylgruppe verknüpft, während bei den neuen Verbindungen ein heterocyclischer Ring, nämlich Furan, in 1-Stellung direkt an den Isochinolinring gebunden ist.

Aufgrund dieser Wirkungen kommen die Verbindungen der allgemeinen Formel I als Wirkstoffe für Arzneimittel zur Behandlung von Durchblutungs- und Coronarstörungen in Frage. Die erfindungsgemäßen Wirkstoffe können enteral oder auch parenteral angewandt werden. Die Dosierung für die orale Anwendung liegt zwischen 0,05 und 50 mg pro kg, bei i. v.-Gabe zwischen 0,01 und 10 mg, vorzugsweise 1 bis 5 mg/kg.

Die neuen 1-Furyl-3,4-dihydroisochinoline der allgemeinen Formel 1 bzw. ihre Säureadditionssalze können auch mit andersartigen Wirkstoffen kombiniert werden. Geeignete galenische Darreichungsformen sind beispielsweise Tabletten., Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung.

## Vorprodukte

### 1. Furan-3,4-dicarbonsäuremonoethylester

Man rührte eine Lösung von 235 g Furan-3,4-dicarbonsäurediethylester und 62,1 g Kaliumhydroxid in 800 ml 60%igem Ethanol 2 Stunden bei Raumtemperatur und destillierte überschüsssiges Lösungsmittel im Vakuum ab. Unter Rühren wurde durch vorsichtiges Zutropfen einer $KHSO_4$-Lösung angesäuert, der Halbester mit $CH_2Cl_2$ extrahiert und aus Essigester/Petroläther kristallisiert.

Ausbeute: 169,3 g (= 83% der Theorie).

2. Furan-3,4-dicarbonsäuremonoethylesterchlorid

183 g Furan-3,4-dicarbonsäuremonoethylester werden zusammen mit 119g Thionylchlorid im Wasserbad 90 min unter Rühren und Rückfluß erhitzt. Überschüssiges Thionylchlorid wird im Vakuum abgezogen, der Rückstand unter vermindertem Druck (147–148°/16 mmHg) destilliert, wobei 183,4 g (91% der Theorie) an Halbesterchlorid erhalten werden.

3. 3-[(3,4-Dimethoxyphenyl)-2-ethylaminocarbonyl]-furan-4-carbonsäureethylester

295 g Furan-3,4-dicarbonsäuremonoethylesterchlorid in 500 ml absolutes Tetrahydrofuran werden bei Raumtemperatur unter Rühren in eine gekühlte Mischung von 263 g 2-(3,4-Dimethoxyphenyl)-ethylamin, 171 g Triethylamin und 500 ml absolutes Tetrahydrofuran eingetropft. Nach beendeter Umsetzung wird ausgefallenes Triethylammoniumchlorid abgesaugt, das Filtrat eingeengt, der feste Rückstand zwischen Wasser und Methylenchlorid verteilt. Die organische Phase wird über $Na_2SO_4$ getrocknet. Nach dem Abdampfen des Lösungsmittels wird aus Essigester umkristallisiert.
Ausbeute: 459 g (= 91% der Theorie); Fp.: 90–92°C.

4. 3-[(3,4-Dimethoxyphenyl)-2-ethylaminocarbonyl]-furan-4-carbonsäure

31,9 g 3-[(3,4-Dimethoxyphenyl)-2-ethylaminocarbonyl]-furan-4-carbonsäureethylester werden in 150 ml Methanol gelöst und mit 150 ml 1 N Natronlauge 45 min bei 50°C gerührt. Danach wird neutralisiert, überschüssiges Lösungsmittel abdestilliert, das Halbamid mit Methylenchlorid extrahiert und aus Essigester-Petroläther kristallisiert.
Ausbeute: 25,5 g (= 87,3% der Theorie); Fp.: 188–189°C.

5. 3-[(3,4-Dimethoxyphenyl)-2-ethylaminocarbonyl]-furan-4-carbonsäure-n-propylamid

Variante A

Eine Lösung von 30 g 3-[(3,4-Dimethoxyphenyl)-2-ethylaminocarbonyl]-furan-4-carbonsäure und 16,2 g N,N'-Carbonyldiimidazol in 150 ml absolutes Tetrahydrofuran wird unter Feuchtigkeitsausschuß 1 Stunde bei Raumtemperatur gerührt. Danach werden 7 ml n-Propylamin hinzugegeben und weitere 4 Stunden bei Raumtemperatur gerührt. Nach beendeter Umsetzung wird das Lösungsmittel im Vakuum abgezogen, der Rückstand in Methylenchlorid gelöst und nacheinander mit verdünnter Salzsäure und Wasser gewaschen. Die organische Phase wird über $Na_2SO_4$ getrocknet, das Lösungsmittel entfernt und der Rückstand aus Essigester kristallisiert.
Ausbeute: 29,4 g (= 87% der Theorie).

Variante B

63 g [3,4-Dimethoxyphenyl)-2-ethylaminocarbonyl]-furan-4-carbonsäureethylester und 120 ml n-Propylamin werden im Autoklaven 24 Stunden auf 120° erhitzt. Das Reaktionsgemisch wird mit 300 ml Ethanol versetzt und bei Siedehitze mit Aktivkohle behandelt. Nach dem Abkühlen wird abgesaugt, eingedampft und der Rückstand aus Essigester/Petroläther kristallisiert. Wenn nötig wird zuvor über eine Kieselgelsäure ($CH_2Cl_2$: MeOH = 100:1 → 100:2) gereinigt.
Ausbeute: 51 g (= 78% der Theorie).

Herstellungsbeispiele

Beispiel 1

3-[3,4-Dihydro-6,7-dimethoxy-isochinolyl-(1)]-furan-4-carbonsäure-ethylester

50 g 3-Carbethoxy-furan-4-carbonsäure-[2-(3,4-dimethoxyphenyl)-ethyl]-amid werden in 120 ml Acetonitril gelöst und mit 18 ml Phosphoroxichlorid versetzt. Das Reaktionsgemisch wird ca. 2 Stunden auf Rückflußtemperatur gehalten. Danach wird eingeengt, der Rückstand in 200 ml Methylenchlorid aufgenommen und durch Einrühren in eine Eiswasser-Pottasche-Lösung alkalisch gestellt. Nach der üblichen Aufarbeitung – Ausschütteln mit Methylenchlorid, Trocknen der organischen Phase über $Na_2SO_4$, Abziehen des Lösungsmittels – wird über eine Kieselgelsäule ($CH_2Cl_2$: MeOH = 100:1 → 100:2 gereinigt.
Ausbeute: 37,5 g (= 79% der Theorie); Fp.: 151–153°C.

Beispiel 2

3-[(3,4-Dihydro-6,7-dimethoxy-isochinolyl-(1)]-furan-4-carbonsäure-hydrochlorid

Zur Hydrolyse werden 15 g 3-[(3,4-Dihydro-6,7-dimethoxy-isochinolyl-(1)]-furan-4-carbonsäure-ethylester in 50 ml Ethanol gelöst und bei 50°C mit 45 ml Kalilauge versetzt. Nach etwa 60 Minuten wurde neutralisiert, das Lösungsmittel abdestilliert, der trockene Rückstand in Ethanol aufgenommen, mit alkoholischer Salzsäure versetzt und abgesaugt. Das Filtrat wird eingeengt und mit Aceton zur Kristallisation gebracht. Nach dem Umkristallisieren aus Methanol/Ether, gegebenenfalls unter Zuhilfenahme von Aktivkohle, verbleiben 13,6 g (= 88,4% der Theorie) reines Produkt. Fp.: 212—214°C.

Beispiel 3

3-[3,4-Dihydro-6,7-dimethoxy-isochinolyl-(1)]-furan-4-carbonsäure-n-propylamid

Variante A

40 g 3-(n-Propylaminocarbonyl)-furan-4-carbonsäure-[2-(3,4-dimethoxyphenyl)-ethyl]-amid werden in 400 ml Acetonitril gelöst und mit 25 ml Phosphoroxichlorid ca. 2 Stunden am Rückfluß erhitzt. Danach wird eingeengt, der Rückstand in 200 ml Methylenchlorid aufgenommen und durch Einrühren in eine Eiswasser-Pottasche-Lösung alkalisch gestellt. Es wird wie unter Beispiel 1 beschrieben aufgearbeitet und über eine Kieselgelsäule ($CH_2Cl_2$: MeOH = 100:1 → 100:2) gereinigt.

Zur Darstellung des Hydrochlorids wird die Base in möglichst wenig Äthanol gelöst und mit äthanolischer Salzsäure versetzt. Das Hydrochlorid wird durch tropfenweisen Zusatz von absolutem Äther zur Kristallisation gebracht.

Ausbeute: 24,1 g (= 57% der Theorie); Fp.: 199—205°C (Z).

Variante B

30,1 g 3-[3,4-Dihydro-6,7-dimethoxy-isochinolyl-(1)]-furan-4-carbonsäure werden in 60 ml Thionylchlorid 40 min am Rückfluß erhitzt. Danach wird überschüssiges Thionylchlorid im Vakuum abgezogen und unter Rühren portionsweise in eine eisgekühlte Lösung von 13 g n-Propylamin in 200 ml absolutem Tetrahydrofuran eingebracht. Nach etwa 1 Stunde wird wie üblich aufgearbeitet, das Umsetzungsprodukt über eine Kieselgelsäule gereinigt und als Hydrochlorid aus MeOH/Ether kristallisiert.

Ausbeute: 16,8 g(= 44,5% der Theorie); Fp.: 204—205°C.

Variante C

33,8 g 3-[3,4-Dihydro-6,7-dimethoxy-isochinolyl-(1)]-furan-4-carbonsäure und 17,2 g N,N'-Carbonyldiimidazol werden bei Rückflußtemperatur in 150 ml absolutem Tetrahydrofuran gelöst und nach 1 Stunde mit 7 ml n-Propylamin versetzt. Man rührt über Nacht bei Raumtemperatur, zieht dann das Lösungsmittel im Vakuum ab, und verteilt den Rückstand zwischen Methylenchlorid und Wasser. Die organische Phase wird getrocknet und eingedampft; der Rückstand über eine Kieselgelsäule gereinigt und als Hydrochlorid aus MeOH/Ether kristallisiert.

Ausbeute: 7,5 g (= 15% der Theorie); Fp.: 203—204°C.

Beispiel 4

3-[3,4-Dihydro-6,7-dimethoxy-isochinolyl-(1)]-furan-4-carbonsäurenitril

10 g 3-[2-(3,4-Dimethoxyphenyl)-ethylaminocarbonyl]-furan-4-carbonsäureamid werden analog Beispiel 1 in 50 ml Acetonitril 1,5 Stunden mit 30 ml Phosphoroxichlorid auf Rückflußtemperatur gehalten. Nach üblicher Aufarbeitung und Reinigung über eine Kieselgelsäule wurde das Hydrochlorid aus Methanol/Ether kristallisiert.

Ausbeute: 7,8 g (= 77% der Theorie); Fp.: 204—207°C.

Analog den vorstehend beschriebenen Beispielen wurden die folgenden Verbindungen der allgemeinen Formel I hergestellt:

| Beispiel | $R_1$ | $R_2$ | $R_3$ | Fp. (°C) | Salzform |
|---|---|---|---|---|---|
| 5 | $CH_3O$ | OH | $COOC_2H_5$ | 173–175 | Base |
| 6 | $CH_3O$ | $CH_3O$ | $CONHCH_3$ | 246 | HCl |
| 7 | $CH_3O$ | $CH_3O$ | $CON(CH_3)_2$ | 167–168 (Z) | |
| 8 | $CH_3O$ | $CH_3O$ | $CONHCH_2CH_3$ | 212–215 (Z) | HCl |
| 9 | $CH_3O$ | $CH_3O$ | $CON(CH_2CH_3)_2$ | 203–205 (Z) | HCl |
| 10 | $CH_3O$ | $CH_3O$ | $CONH(CH_2)_3CH_3$ | 216 (Z) | HCl |
| 11 | $CH_3O$ | $CH_3O$ | $CONH(CH_2)_4CH_3$ | 215–218 (Z) | HCl |
| 12 | $CH_3O$ | $CH_3O$ | $CONHCH_2CH(CH_3)_2$ | 195–200 (Z) | HCl |
| 13 | $CH_3O$ | $CH_3O$ | $CONHCH_2CH_2CH(CH_3)_2$ | 222–225 (Z) | HCl |
| 14 | $CH_3O$ | $CH_3O$ | $CONH—CH_2—C{\equiv}CH$ | 244–245 (Z) | HCl |
| 15 | $CH_3O$ | $CH_3O$ | $CONH(CH_2)_2B(CH_3)_2$ | 162–165 | HCl |
| 16 | $CH_3O$ | $CH_3O$ | $CONH(CH_2)_3N(CH_3)_2$ | 116–125 (Z) | HCl |
| 17 | $CH_3O$ | $CH_3O$ | $CONH—CH_2CH_2OCH_3$ | 216–218 (Z) | HCl |
| 18 | $CH_3O$ | $CH_3O$ | $CONH—CH_2—CH_2—CH_2Cl$ | 233–237 (Z) | HCl |
| 19 | $CH_3O$ | $CH_3O$ | $CO—NH—CH_2—CH(OH)—CH_3$ | 218–224 (Z) | HCl |
| 20 | $CH_3O$ | $CH_3O$ | CO— 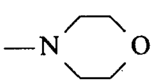 | 141–144 | HCl |
| 21 | $CH_3O$ | $CH_3O$ | $CO—NH—CH_2CH_2—$ 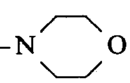 | 183–185 | HCl |
| 22 | $CH_3O$ | $CH_3O$ | $CONH—CH_2CH_2—$ 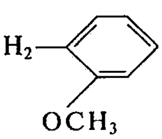 | 221–222 | HCl |
| 23 | $CH_3O$ | $CH_3O$ | $CO—NH—CH_2—CH_2—$ 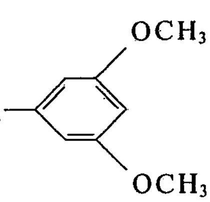 | 194–198 | HCl |
| 24 | $CH_3O$ | $CH_3O$ | $CO—NH—CH_2—CH_2—$ 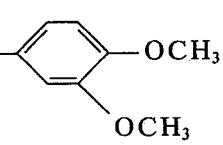 | 186–192 | HCl |
| 25 | $CH_3O$ | $CH_3O$ | $CO—NH—CH_2—$ 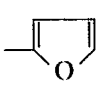 | 231–234 | HCl |

## Formulierungsbeispiele

### Beispiel A: Dragées

| | | |
|---|---|---|
| Wirkstoff gemäß Erfindung | | 5 mg |
| Milchzucker | | 65 mg |
| Maisstärke | | 130 mg |
| sek. Calciumphosphat | | 40 mg |
| lösliche Stärke | | 3 mg |
| Magnesiumstearat | | 3 mg |
| kolloidale Kieselsäure | | 4 mg |
| | insgesamt | 250 mg |

### Herstellung

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht gepreßt, die dann in üblicher Weise mit Hilfe von Zucker, Talkum und Gummi arabicum dragiert werden.

### Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 ml |

### Herstellung

Wirkstoff und Natriumchlorid werden in Wasser gelöst und unter Stickstoff in Glasampullen abgefüllt.

### Beispiel C: Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

**Patentansprüche**

1. 1-Furyl-3,4-dihydroisochinoline der allgemeinen Formel I

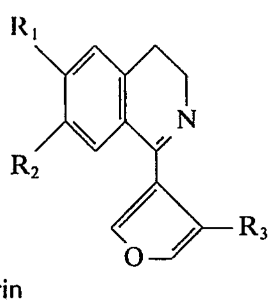

(I)

worin

$R_1$ eine Methoxygruppe,
$R_2$ eine Methoxy- oder Hydroxygruppe,
$R_3$ eine Cyanogruppe oder eine

$-\underset{\underset{O}{\|}}{C}-Y$-Gruppe

bedeutet, wobei

Y —OH, —O-Alkyl mit 1 bis 3 Kohlenstoffatomen,

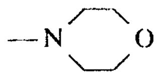, $-N(CH_3)_2$, $-N(C_2H_5)_2$,

—NH-Alkyl mit 1 bis 5 Kohlenstoffatomen,
$-NH-CH_2-CHOH-CH_3$, $-NH-CH_2-C{\equiv}CH$

oder eine Gruppe der Teilformel

$-NH-(CH_2)_n-R$

bedeutet, in der

n die Zahlen 1 bis 3 und
R $-N(CH_3)_2$, $-OCH_3$, $-Cl$,

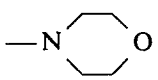, 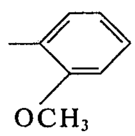, 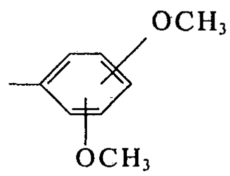 oder 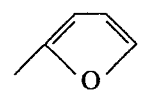

bezeichnen und deren Säureadditionssalze.

2. 1-Furyl-3,4-dihydroisochinoline nach Anspruch 1 der allgemeinen Formel

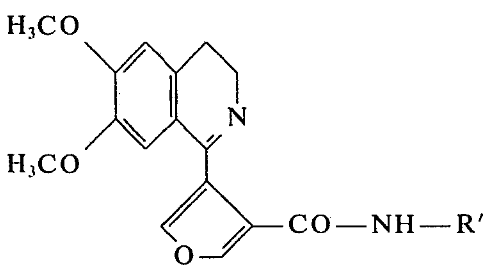

(Ia)

worin R' eine Alkylgruppe mit 1 bis 5 Kohlenstoffatomen, eine Dialkylaminoalkylgruppe oder den Rest —CN bedeutet und deren Säureadditionssalze.

3. 1-Furyl-3,4-dihydroisochinoline nach Anspruch 2 der allgemeinen Formel Ia, worin R' eine Alkylgruppe der Teilformel

$-(CH_2)_2-CH_3$,
$-(CH_2)_3-CH_3$ oder
$-CH_2-CH(CH_3)_2$

bedeutet und deren Säureadditionssalze.

4. Verfahren zur Herstellung von Verbindungen der allgemeinen Formeln I oder Ia nach den Ansprüchen 1 bis 3, dadurch gekennzeichnet, daß man

a) ein Amid der Furan-3,4-dicarbonsäure der allgemeinen Formel

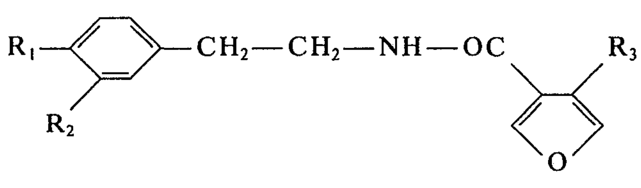

(II)

worin $R_1$ bis $R_3$ die oben genannten Bedeutungen besitzen, mit Lewissäuren cyclisiert; oder

b) ein 3-[(3,4-Dihydroisochinolyl-(1)]-furan-4-carbonsäurederivat der allgemeinen Formel

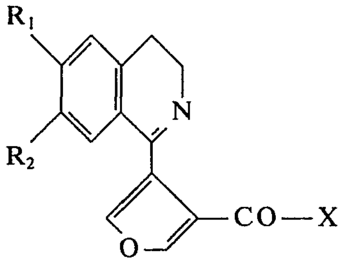

(III)

worin $R_1$ und $R_2$ die oben genannten Bedeutungen besitzen und X ein Chloratom oder eine Methoxy- oder Ethoxygruppe oder eine Imidazolidgruppe bedeutet, mit einer Verbindung der allgemeinen Formel

Y–H (IV)

worin Y die in Anspruch 1 angegebene Bedeutung besitzt, umsetzt und die erhaltene Verbindung gegebenenfalls in ein Säureadditionssalz überführt.

5. Verfahren nach Anspruch 4a, dadurch gekennzeichnet, daß man die Cyclisierung mit Phosphortrichlorid, Phosphorpentachlorid, Phosphoroxychlorid, Bortrifluorid, Zinntetrachlorid, Schwefelsäure, Fluorsulfonsäure oder Fluorwasserstoffsäure durchführt.

6. Verfahren nach Anspruch 4a und 5, dadurch gekennzeichnet, daß man die Cyclisierung in Gegenwart eines inerten Lösungsmittels bei Siedetemperatur des Lösungsmittels durchführt.

7. Pharmazeutische Zubereitungen, dadurch gekennzeichnet, daß sie eine oder mehrere Verbindungen nach einem der Ansprüche 1 bis 3 neben üblichen Hilfs- und Trägerstoffen enthalten.

8. Verfahren zur Herstellung pharmazeutischer Zubereitungen nach Anspruch 7, dadurch gekennzeichnet, daß man eine oder mehrere Verbindungen nach einem der Ansprüche 1 bis 3 mit üblichen Hilfs-, Träger-, Spreng- oder Schmiermitteln bzw. Substanzen zur Erzielung einer Depotwirkung zu Tabletten, Kapseln, Zäpfchen, Pulver oder Lösungen formuliert.

9. Verbindungen nach einem der Ansprüche 1 bis 3 zur Verwendung bei der Behandlung von Durchblutungsstörungen und Coronarerkrankungen.

**Claims**

1. 1-Furyl-3,4-dihydroisoquinolines of general formula

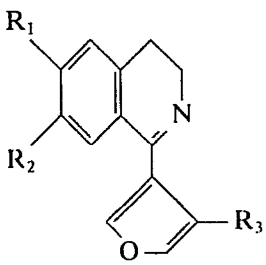

(I)

wherein

$R_1$ represents a methoxy group,
$R_2$ represents a methoxy or hydroxy group,
$R_3$ represents a cyano group or a

—C—Y-group
||
O

wherein

Y represents —OH, —O-alkyl with 1 to 3 carbon atoms,

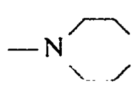 O, $-N(CH_3)_2$, $-N(C_2H_5)_2$,

$-NH$-alkyl with 1 to 5 carbon atoms, $-NH-CH_2-CHOH-CH_3$, $-NH-CH_2-C{\equiv}CH$

or a group with the partial formula

$-NH-(CH_2)_n-R$

wherein

n represents the numbers 1 to 3 and
R represents $-N(CH_3)_2$, $-OCH_3$, $-Cl$,

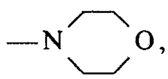, 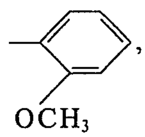, 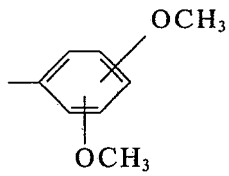 or 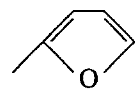

and the acid addition salts thereof.

2. 1-Furyl-3,4-dihydroisoquinolines as claimed in claim 1 of general formula

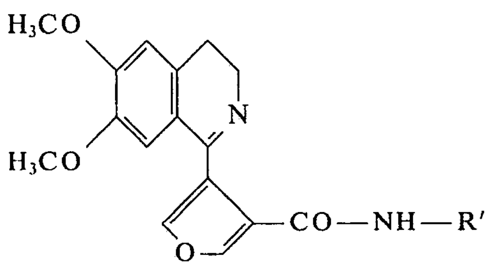

(Ia)

wherein R' represents an alkyl group with 1 to 5 carbon atoms, a dialkylaminoalkyl group or the group $-CN$ and the acid addition salts thereof.

3. 1-Furyl-3,4-dihydroisoquinolines as claimed in claim 2 of general formula Ia, wherein R' represents an alkyl group with the partial formula

$-(CH_2)_2-CH_3$,
$-(CH_2)_3-CH_3$ or
$-CH_2-CH(CH_3)_2$

and the acid addition salts thereof.

4. Process for preparing compounds of general formula I or Ia as claimed in claim 1 to 3, characterised in that

a) an amide of furan-3,4-dicarboxylic acid of general formula

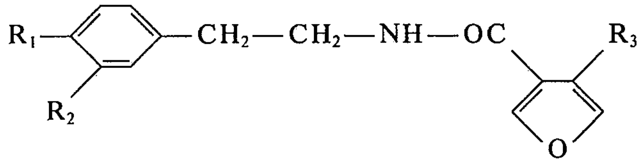

(II)

wherein $R_1$ to $R_3$ are as hereinbefore defined, is cyclised with Lewis acids; or

b) a 3-[(3,4-dihydroisoquinolyl-(1)]-furan-4-carboxylic acid derivative of general formula

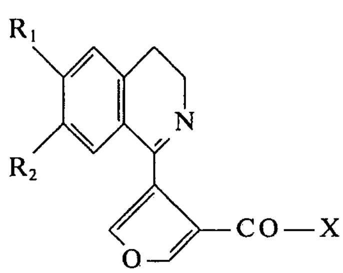

(III)

wherein $R_1$ and $R_2$ are as hereinbefore defined and X represents a chlorine atom or a methoxy or ethoxy group or an imidazolide group, is reacted with a compound of general formula IV

$$Y-H \qquad (IV)$$

wherein Y is as defined in claim 1, and the resulting compound is optionally converted into an acid addition salt.

5. Process as claimed in claim 4a, characterised in that the cyclisation is effected with phosphorus trichloride, phosphorus pentachloride, phosphorus oxychloride, boron trifluoride, tin tetrachloride, sulphuric acid, fluorosulphonic acid or hydrofluoric acid.

6. Process as claimed in claims 4a and 5, characterised in that the cyclisation is effected in the presence of an inert solvent at the boiling temperature of the solvent.

7. Pharmaceutical preparations, characterised in that they contain one or more compounds as claimed in one of claims 1 to 3 together with conventional excipients and carriers.

8. Process for preparing pharmaceutical preparations as claimed in claim 7, characterised in that one or more compounds as claimed in one of claims 1 to 3 are processed with conventional exipients, carriers, disintegrants or lubricants or substances for obtaining delayed release, to form tablets, capsules, suppositories, powder or solutions.

9. Compounds as claimed in one of claim 1 to 3 for use in the treatment of circulatory disorders and coronary diseases.

**Revendications**

1. 1-furyl-3,4-dihydroisoquinoléines de formule générale I,

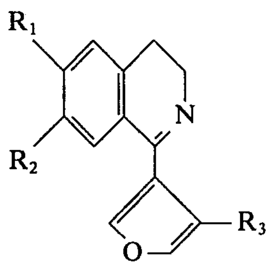

(I)

dans laquelle

$R_1$ désigne un groupe méthoxy,
$R_2$ désigne un groupe méthoxy ou hydroxy,
$R_3$ désigne un groupe cyano ou un groupe

$$\underset{\displaystyle O}{\overset{}{\underset{\|}{-C}}}-Y$$

où

Y représente –OH, –O-alcoyle avec 1 à 3 atomes de carbone,

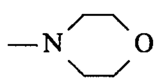 , $-N(CH_3)_2$, $-N(C_2H_5)_2$,

–NH-alcoyle avec 1 à 5 atomes de carbone,
$-NH-CH_2-CHOH-CH_3$, $-NH-CH_2-C{\equiv}CH$

ou un groupe de formule partielle

$-NH-(CH_2)_n-R$,

dans laquelle

n désigne les nombres 1 à 3 et
R désigne $-N(CH_3)_2$, $-OCH_3$, –Cl,

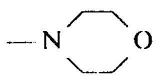, 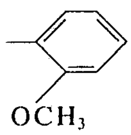, 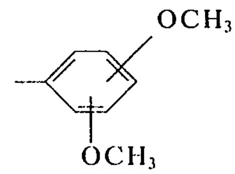 ou 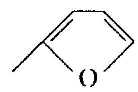

et leurs sels d'addition d'acides.

2. 1-furyl-3,4-dihydroisoquinoléines selon la revendication 1 de formule générale

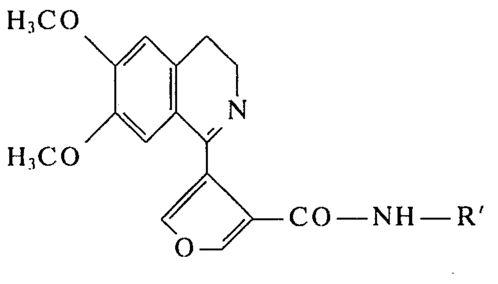

(Ia)

dans laquelle R' représente un groupe alcoyle avec 1 à 5 atomes de carbone, un groupe dialcoylaminoalcoyle ou le radical —CN et leurs sels d'addition d'acides.

3. 1-furyl-3,4-dihydroisoquinoléines selon la revendication 2 de formule générale Ia, dans laquelle R' représente un groupe alcoyle de formule partielle

$-(CH_2)_2-CH_3$,
$-(CH_2)_3-CH_3$ ou
$-CH_2-CH(CH_3)_2$

et leurs sels d'addition d'acides.

4. Procédé pour la préparation de composés de formules générales I ou Ia selon les revendications 1 à 3, caractérisé en ce que

a) on cyclise un amide de l'acide furanne-3,4-dicarboxylique de formule générale

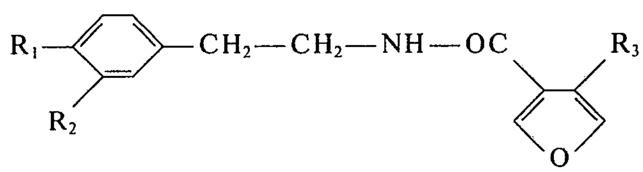

(II)

dans laquelle $R_1$ à $R_2$ possèdent les significations mentionnées plus haut, au moyen d'acides le Lewis; ou

b) on fait réagir un dérivé d'acide 3-[(3,4-dihydroisoquinoléyl-(1)]-furanne-4-carboxylique de formule générale

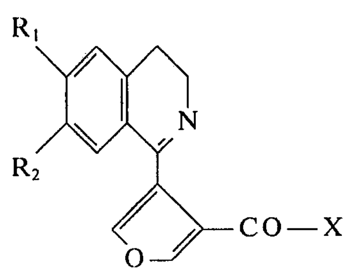

(III)

dans laquelle $R_1$ et $R_2$ possèdent les significations mentionnées plus haut et X représente un atome de chlore ou un groupe méthoxy ou éthoxy ou un groupe imidazolide, avec un composé de formule générale

Y—H (IV)

dans laquelle Y possède la signification indiquée dans la revendication 1 et on transforme éventuellement le composé obtenu en un sel d'addition d'acide.

5. Procédé selon la revendication 4a, caractérisé en ce qu'on effectue la cyclisation avec du trichlorure de phosphore, du pentachlorure de phosphore, de l'oxychlorure de phosphore, du trifluorure de bore, du tétrachlorure d'étain, de l'acide sulfurique, de l'acide fluorsulfonique ou de l'acide fluorhydrique.

6. Procédé selon la revendication 4a et 5, caractérisé en ce qu'on effectue la cyclisation en présence d'un solvant inerte à la température d'ébullition du solvant.

7. Préparations pharmaceutiques, caractérisées en ce qu'elles contiennent un ou plusieurs composés selon l'une des revendications 1 à 3 conjointement à des adjuvants et excipients usuels.

8. Procédé pour la préparation de compositions pharmaceutiques selon la revendication 7, caractérisé en qu'on formule un ou plusieurs composés selon l'une des revendications 1 à 3 avec des adjuvants, excipients, désagrégeants ou lubrifiants ou respectivement substaces pour obtenir un effet retard usuels en comprimés, capsules, suppositoires, poudres ou solutions.

9. Composés selon l'une des revendications 1 à 3 pour l'utilisation dans le traitement des perturbations de la circulation sanguine et des maladies des coronaires.